# EUROPEAN PATENT APPLICATION

(11) **EP 4 714 957 A1**
(43) Date of publication of application: **25.03.2026**
(21) Application number: 24807132.6
(22) Date of filing: 10.05.2024
(51) Int. Cl.: C07D 493/10, C07C 51/50, C07C 67/03, C07C 67/62, C08F 2/00

(54) **(METH)ACRYLATE COMPOUND PRODUCTION METHOD**

(30) Priority: 16.05.2023 JP 2023080939
(71) Applicant: MITSUBISHI GAS CHEMICAL COMPANY, INC., Chiyoda-ku Tokyo 100-8324 (JP)
(72) Inventor: NAKAKURA, Koki, Kurashiki-shi, Okayama 712-8525 (JP); SHIRAI, Shinyou, Kurashiki-shi, Okayama 712-8525 (JP)
(74) Representative: Müller-Boré & Partner Patentanwälte PartG mbB
(86) International application number: PCT/JP2024/017342
(87) International publication number: WO 2024/237180

(57) **Abstract**

The present invention provides, for example, a method for producing a (meth)acrylate compound which suppresses the polymerization of a starting material acrylic acid ester during synthesis and is capable of producing a more highly pure reaction product. The method for producing a (meth)acrylate compound according to the present invention comprises at least the step of performing transesterification reaction of spiroglycol and a (meth)acrylic acid ester in the presence of a polymerization inhibitor, wherein the polymerization inhibitor comprises an N-oxyl compound and a polymerization inhibitor having a boiling point of 246°C or lower.

## Description

### Technical Field

The present invention relates to, for example, a method for producing a (meth)acrylate compound.

### Background Art

(Meth)acrylate compounds having a spiroacetal backbone in a molecule are excellent in mechanical strength, heat resistance, moisture resistance, transparency, and the like and as such, are compounds that are useful as intermediates or monomers of polymer materials such as polycarbonate, polyester, polyester carbonate, polyacrylate, polyurethane, polyether polyol, and epoxy resin, and further as starting materials for coating materials, ink, adhesives, plasticizers, lubricating oils, films, sheets, and the like. Also, these compounds are widely used as polymerizable compounds for thermosetting resin compositions, photocurable resin compositions, ionizing radiation-curable ink compositions, hologram compositions, and the like, or as additives for compositions for lithium ion-conducting electrolytes in a wide range of fields, for example, optimal members such as optical lenses, illumination members, electronic materials, and automobile members.

For example, a dehydration condensation method of performing dehydrative esterification reaction of spiroglycol and (meth)acrylic acid (see Patent Literatures 1 to 3), a transesterification method of performing dealcoholization and esterification reaction of spiroglycol and ethyl acrylate (see Patent Literature 4), and a transesterification method of performing dealcoholization and esterification reaction of spiroglycol and methyl (meth)acrylate (see Patent Literature 5) are known as methods for producing a (meth)acrylate compound having a spiroacetal backbone in a molecule.

### Citation List

### Patent Literature

Patent Literature 1: Japanese Patent Laid-Open No. S59-078193
Patent Literature 2: Japanese Patent Laid-Open No. S60-142990
Patent Literature 3: Japanese Patent Laid-Open No. 2005-343816
Patent Literature 4: Japanese Patent Laid-Open No. S57-170933
Patent Literature 5: Japanese Patent Laid-Open No. 2006-169374

### Summary of Invention

### Technical Problem

However, the dehydration condensation methods of Patent Literatures 1 to 3, which involve performing dehydration esterification reaction of spiroglycol and (meth)acrylic acid, have produced reaction products with a very low purity. This is presumably because a hydrolysate of spiroglycol, for example, results from water generated during synthesis in the presence of a strong acid catalyst such as p-toluenesulfonic acid, and further, the hydrolysate forms a secondary product or the like with the (meth)acrylic acid and thereby increases the amount of impurities. Reaction products obtained by the dehydration condensation methods of Patent Literatures 1 to 3 thereby have the difficulty in being used as starting materials for highly functional polymers or highly functional materials.

In order to circumvent this problem, studies have been pursued not on the dehydration condensation, which causes water to be generated during reaction, but on dealcoholization transesterification of spiroglycol and a (meth)acrylic acid ester. However, the production methods of Patent Literatures 4 and 5 have been found to cause a starting material acrylic acid ester to be polymerized during synthesis, which is problematic.

The present invention has been made in light of these problems, and an object of the present invention is to provide, for example, a method for producing a (meth)acrylate compound which suppresses the polymerization of a starting material acrylic acid ester during synthesis and is capable of producing a more highly pure reaction product.

### Solution to Problem

The present inventors have conducted diligent studies and consequently completed the present invention by finding that the object can be attained by performing transesterification reaction of spiroglycol and a (meth)acrylic acid ester in the presence of a predetermined polymerization inhibitor.

Specifically, the present invention provides various embodiments given below.
[1] A method for producing a (meth)acrylate compound, comprising at least the step of
   performing transesterification reaction of spiroglycol and a (meth)acrylic acid ester in the presence of a polymerization inhibitor, wherein
   the polymerization inhibitor comprises an N-oxyl compound and a polymerization inhibitor having a boiling point of 246°C or lower.
[2] The method for producing a (meth)acrylate compound according to [1], wherein
   in the step of performing the transesterification reaction, the transesterification reaction is performed in the absence of a solvent other than the (meth)acrylic acid ester.
[3] The method for producing a (meth)acrylate compound according to [1] or [2], wherein
   in the step of performing the transesterification reaction, a content ratio of the N-oxyl compound is 5 ppm or more and 300 ppm or less based on the total amount of the spiroglycol and the (meth)acrylic acid ester.
[4] The method for producing a (meth)acrylate compound according to any one of [1] to [3], wherein
   in the step of performing the transesterification reaction, a content ratio of the polymerization inhibitor having a boiling point of 246°C or lower is 10 ppm or more and 3000 ppm or less based on the total amount of the spiroglycol and the (meth)acrylic acid ester.
[5] The method for producing a (meth)acrylate compound according to any one of [1] to [4], wherein
   the polymerization inhibitor having a boiling point of 246°C or lower comprises a phenolic compound and/or a quinone compound.
[6] The method for producing a (meth)acrylate compound according to any one of [1] to [5], wherein
   the polymerization inhibitor having a boiling point of 246°C or lower comprises hydroquinone monomethyl ether.
[7] The method for producing a (meth)acrylate compound according to any one of [1] to [6], wherein
   the N-oxyl compound is one or more selected from the group consisting of 4-hydroxy-2,2,6,6-tetramethylpiperidine-1-oxyl, 4-oxo-2,2,6,6-tetramethylpiperidine-1-oxyl, 4-methoxy-2,2,6,6-tetramethylpiperidine-1-oxyl, 4-cyano-2,2,6,6-tetramethylpiperidine-1-oxyl, 4-amino-2,2,6,6-tetramethylpiperidine-1-oxyl, 4-carboxy-2,2,6,6-tetramethylpiperidine-1-oxyl, 4-methacryloyloxy-2,2,6,6-tetramethylpiperidine-1-oxyl, 4-acetamido-2,2,6,6-tetramethylpiperidine-1-oxyl, 4-maleimido-2,2,6,6-tetramethylpiperidine-1-oxyl, 4-phosphonooxy-2,2,6,6-tetramethylpiperidine-1-oxyl, 4-maleimido-2,2,6,6-tetramethylpiperidine-1-oxyl, pyrrolidine-1-oxyl free radical compounds, 3-carboxy-2,2,5,5-tetramethylpyrrolidine-1-oxyl, and 2,2,6,6-tetramethylpiperidine-1-oxyl.
[8] The method for producing a (meth)acrylate compound according to any one of [1] to [7], wherein
   the (meth)acrylic acid ester is a (meth)acrylic acid alkyl ester.

### Advantageous Effect of Invention

The present invention can provide, for example, a method for producing a (meth)acrylate compound which suppresses the polymerization of a starting material acrylic acid ester during synthesis and is capable of producing a more highly pure reaction product.

### Description of Embodiments

Hereinafter, the mode for carrying out the present invention (hereinafter, referred to as the "present embodiment") will be described in detail. In this context, the embodiments described below are illustrations for describing the present invention, and the present invention is not limited by these embodiments. Specifically, the present invention can be carried out through arbitrary changes or modifications made without departing from the spirit of the present invention. In the present specification, for example, the notation of the numeric range of "1 to 100" means a numeric range including both the lower limit value "1" and the upper limit value "100". The same holds true for the notation of other numeric ranges.

In the present specification, the (meth)acrylic acid refers to both or any one of acrylic acid and methacrylic acid, and the (meth)acrylate refers to both or any one of acrylate and methacrylate.

### [Method for producing (meth)acrylate compound]

The method for producing a (meth)acrylate compound according to the present embodiment comprises at least the step of performing transesterification reaction of spiroglycol and a (meth)acrylic acid ester in the presence of a polymerization inhibitor, wherein the polymerization inhibitor comprises an N-oxyl compound and a polymerization inhibitor having a boiling point of 246°C or lower. Use of the N-oxyl compound and the polymerization inhibitor having a boiling point of 246°C or lower in combination as the polymerization inhibitor can suppress the polymerization of a starting material acrylic acid ester, which occurs unintentionally during transesterification reaction, and can thereby produce a highly pure reaction product.

The spiroglycol for use as a starting material 1 is 3,9-bis(2-hydroxy-1,1-dimethylethyl)-2,4,8,10-tetraoxaspiro[5.5]undecane represented by the following formula:

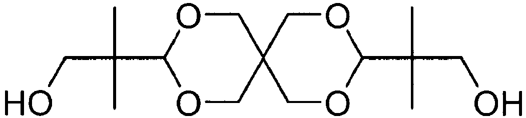

The (meth)acrylic acid ester for use as a starting material 2 is preferably a (meth)acrylic acid alkyl ester from the viewpoint of performing the transesterification reaction by dealcoholization. In this context, the alkyl ester site is preferably an alkyl group having 1 to 6 carbon atoms, more preferably an alkyl group having 1 to 4 carbon atoms, further preferably an alkyl group having 1 to 3 carbon atoms, particularly preferably an alkyl group having 1 or 2 carbon atoms. Specific examples of the alkyl group include, but are not particularly limited to, a methyl group, an ethyl group, a n-propyl group, an isopropyl group, a butyl group, an isobutyl group, a s-butyl group, a t-butyl group, a cyclohexyl group, and a phenyl group. One (meth)acrylic acid ester may be used alone, or two or more thereof may be used in combination. The (meth)acrylic acid alkyl ester is more preferably methyl (meth)acrylate or ethyl (meth)acrylate.

In the production method of the present embodiment, it is essential to use the (meth)acrylic acid ester as the starting material 2 from the viewpoint of suppressing the secondary production of impurities ascribable to generated water. In other words, the transesterification reaction step preferably involves substantially no (meth)acrylic acid. In this context, the phrase "involve substantially no (meth)acrylic acid" means less than 10,000 ppm, preferably less than 5,000 ppm, more preferably less than 1,000 ppm, further preferably less than 500 ppm, based on the total amount of the spiroglycol and the (meth)acrylic acid ester charged in the transesterification reaction step. The lower limit of the content ratio of the (meth)acrylic acid is preferably 0 ppm or equal to or less than a measurement limit, as a matter of course.

In the production method of the present embodiment, preferably, spiroglycol di(meth)acrylate is obtained as the (meth)acrylate compound through the transesterification reaction of the spiroglycol and the (meth)acrylic acid alkyl ester mentioned above. In this respect, an alcohol is secondarily produced through dealcoholization reaction.

The transesterification reaction of the spiroglycol and the (meth)acrylic acid alkyl ester mentioned above can be performed in accordance with a conventional method and is not particularly limited. In general, this reaction can be performed under a temperature condition involving a reaction liquid temperature of 65 to 120°C in the presence of a catalyst. The transesterification reaction is preferably performed under a reflux condition of the (meth)acrylic acid ester from the viewpoint of effects mentioned later of two polymerization inhibitors used in combination. Hence, the reaction liquid temperature is preferably 65°C or higher, more preferably 70°C or higher, and preferably 110°C or lower, more preferably 100°C or lower. The reaction pressure in the transesterification reaction is not particularly limited and may be any of ordinary pressure, reduced pressure, and increased pressure. The reaction time in the transesterification reaction is not particularly limited and is preferably 1 to 12 hours from the viewpoint of industrial production efficiency.

The transesterification reaction can be carried out by any of batch and continuous methods. In the case of performing the continuous scheme, a continuous countercurrent contact method is industrially advantageous. As an example of the batch scheme, a reactor is charged with spiroglycol, (meth)acrylic acid ester, a polymerization inhibitor, and optionally a catalyst, and stirred at a predetermined temperature while the reaction liquid is bubbled, if necessary, with an oxygen-containing gas. Then, a monohydric alcohol derived from dealcoholization reaction is secondarily produced as the transesterification reaction progresses. This monohydric alcohol may remain in the reaction system, whereas the progression of the transesterification reaction can be accelerated by discharging the monohydric alcohol to the outside of the reaction system.

In the transesterification reaction, an oxygen-containing gas is preferably introduced into the system from the viewpoint of suppressing the polymerization of the (meth)acrylic acid ester. Specific examples of the oxygen-containing gas include, but are not particularly limited to, air, a mixed gas of oxygen and nitrogen, and a mixed gas of air and nitrogen. Examples of the method for introducing the gas include a method of blowing the gas into a reaction product (so-called bubbling). The internal pressure of the system in bringing the oxygen-containing gas into contact therewith may be any of ordinary pressure, reduced pressure, and increased pressure. The internal temperature of the system in bringing the oxygen-containing gas into contact therewith is not particularly limited and is preferably 65 to 110°C, more preferably 70 to 100°C.

The catalyst for use in the transesterification reaction is not particularly limited, and a transesterification catalyst known in the art can be used. Examples of the catalyst that may be used include: acid catalysts such as organic acids (e.g., paratoluenesulfonic acid and methanesulfonic acid), inorganic acids (e.g., hydrochloric acid, sulfuric acid, nitric acid, and phosphoric acid), and cation exchange resins; metal alkoxide such as alkali metal alkoxide, magnesium alkoxide, aluminum alkoxide, zirconium alkoxide (e.g., zirconium tetrabutoxide), and titanium alkoxide (e.g., tetramethyl titanate, tetrabutyl titanate, tetrapropyl titanate, tetraisopropyl titanate, tetraisobutyl titanate, and tetraphenyl titanate); organic tin compounds such as dibutyltin oxide, dioctyltin oxide, dibutyltin dilaurate, dibutyltin dimethoxide, and dibutyltin diacetate; and anion exchange resins. One of these catalysts may be used alone, or two or more thereof may be used in combination. However, for the production method of the present embodiment, it is preferred to not use Bronsted acid catalysts, azabicyclo compounds such as azabicyclo compounds, amidine compounds, pyridine compounds, and phosphine compounds, or zinc-based catalysts such as organic acid zinc and zinc diketone enolate, from the viewpoint of suppressing the production of a spiroglycol degradation product and a reaction by-product thereof with the (meth)acrylic acid ester and obtaining a more highly pure reaction product. Hence, the catalyst for use in the production method of the present embodiment is preferably metal alkoxide, an organic tin compound, or an anion exchange resin, more preferably metal alkoxide. The amount of the catalyst used can be appropriately set depending on the desired performance and is not particularly limited. The amount is preferably 1:0.001 to 0.2, more preferably 1:0.005 to 0.1, further preferably 1:0.01 to 0.05, in terms of a molar ratio to 1 mol in total of the spiroglycol and the (meth)acrylic acid ester charged in the transesterification reaction step.

Use of a solvent is not essential for the transesterification reaction, and the transesterification reaction can be performed under a condition of the absence of a solvent other than the (meth)acrylic acid ester. However, a solvent may be used, if necessary. In the case of using the solvent, a nonaqueous solvent is preferably used from the viewpoint of suppressing the production of impurities. Specific examples of the nonaqueous solvent include, but are not particularly limited to: ketones such as acetone, methyl ethyl ketone, methyl isobutyl ketone, cyclohexanone, acetophenone, and benzophenone; esters such as methyl benzoate and γ-butyrolactone; carbonate compounds such as dimethyl carbonate, diethyl carbonate, ethylene carbonate, propylene carbonate, and 1,2-butylene carbonate; sulfones such as sulfolane; sulfoxides such as dimethyl sulfoxide; hydrocarbons such as n-hexane, cyclohexane, methylcyclohexane, n-heptane, n-octane, n-nonane, n-decane, benzene, toluene, xylene, ethylbenzene, diethylbenzene, isopropylbenzene, amylbenzene, diamylbenzene, triamylbenzene, dodecylbenzene, didodecylbenzene, amyltoluene, isopropyltoluene, decalin, and tetralin; and ethers such as diethyl ether, dipropyl ether, diisopropyl ether, dibutyl ether, diamyl ether, diethyl acetal, dihexyl acetal, t-butyl methyl ether, cyclopentyl methyl ether, tetrahydrofuran, tetrahydropyran, trioxane, dioxane, anisole, diphenyl ether, dimethyl cellosolve, diglyme, triglyme, and tetraglyme. One of these solvents may be used alone, or two or more thereof may be used in combination. Among them, ketones, ethers, and hydrocarbons are preferred from the viewpoint of removal efficiency under a reflux condition, or the like. In the case of using the solvent, the amount of the solvent used is not particularly limited and is preferably 1 to 70 parts by mass, more preferably 5 to 50 parts by mass, further preferably 10 to 30 parts by mass, based on 100 parts by mass in total of the spiroglycol and the (meth)acrylic acid ester charged in the transesterification reaction step.

In the transesterification reaction, the charging proportions of the spiroglycol and the (meth)acrylic acid ester can be appropriately set depending on the desired performance and are not particularly limited. The charging proportion of the (meth)acrylic acid ester is preferably 1 to 15 times the molar quantity of the spiroglycol from the viewpoint of reaction efficiency in performing the transesterification reaction under a reflux condition of the (meth)acrylic acid ester. The charging proportion is more preferably 5 or more times, further preferably 7 or more times, and more preferably 12 or less times, further preferably 10 or less times the molar quantity thereof.

In the transesterification reaction, an N-oxyl compound and a polymerization inhibitor having a boiling point of 246°C or lower are used in combination as the polymerization inhibitor. The reason is not certain why such use of two polymerization inhibitors in combination can suppress the polymerization of a starting material acrylic acid ester. According to the findings of the present inventors, the following is deduced: in the production method of the present embodiment, the transesterification reaction is performed in the state of spiroglycol dissolved or dispersed in liquid (meth)acrylic acid ester. In this context, the N-oxyl compound suppresses the polymerization of the (meth)acrylic acid ester in a reaction liquid containing the N-oxyl compound. On the other hand, the (meth)acrylic acid ester vaporizes gradually from the reaction liquid with increase in reaction liquid temperature so that a portion of the (meth)acrylic acid ester exists in a vapor phase in a reaction vessel. This vapor phase rarely contains the N-oxyl compound and permits progression of the polymerization of the (meth)acrylic acid ester. Accordingly, in the production method of the present embodiment, use of the polymerization inhibitor having a boiling point of 246°C or lower in combination therewith diffuses the polymerization inhibitor into the vapor phase and thereby suppresses the polymerization of the (meth)acrylic acid ester. However, effects are not limited by this deduction.

In this context, examples of the N-oxyl compound include, but are not particularly limited to, 4-hydroxy-2,2,6,6-tetramethylpiperidine-1-oxyl, 4-oxo-2,2,6,6-tetramethylpiperidine-1-oxyl, 4-methoxy-2,2,6,6-tetramethylpiperidine-1-oxyl, 4-cyano-2,2,6,6-tetramethylpiperidine-1-oxyl, 4-amino-2,2,6,6-tetramethylpiperidine-1-oxyl, 4-carboxy-2,2,6,6-tetramethylpiperidine-1-oxyl, 4-methacryloyloxy-2,2,6,6-tetramethylpiperidine-1-oxyl, 4-acetamido-2,2,6,6-tetramethylpiperidine-1-oxyl, 4-maleimido-2,2,6,6-tetramethylpiperidine-1-oxyl, 4-phosphonooxy-2,2,6,6-tetramethylpiperidine-1-oxyl, 4-maleimido-2,2,6,6-tetramethylpiperidine-1-oxyl, pyrrolidine-1-oxyl free radical compounds, 3-carboxy-2,2,5,5-tetramethylpyrrolidine-1-oxyl, and 2,2,6,6-tetramethylpiperidine-1-oxyl. One of these N-oxyl compounds may be used alone, or two or more thereof may be used in combination.

The polymerization inhibitor having a boiling point of 246°C or lower is preferably a phenolic compound and/or a quinone compound, more preferably hydroquinone monomethyl ether (boiling point: 243°C) or p-benzoquinone (boiling point: up to 180°C/sublimation). Use of such a polymerization inhibitor having a low boiling point or sublimation can diffuse the polymerization inhibitor into the vapor phase and thereby suppress the polymerization of the (meth)acrylic acid ester. One of these polymerization inhibitors having a boiling point of 246°C or lower may be used alone, or two or more thereof may be used in combination.

The usage amounts of the N-oxyl compound and the polymerization inhibitor having a boiling point of 246°C or lower described above can be appropriately set depending on the desired performance and are not particularly limited. In the step of performing the transesterification reaction, the content ratio of the N-oxyl compound is preferably 1 ppm or more and 300 ppm or less, more preferably 3 ppm or more and 200 ppm or less, further preferably 5 ppm or more and 100 ppm or less, based on the total amount of the spiroglycol and the (meth)acrylic acid ester, from the viewpoint of suppressing the polymerization of the (meth)acrylic acid ester. In the step of performing the transesterification reaction, the content ratio of the polymerization inhibitor having a boiling point of 246°C or lower is preferably 10 ppm or more and 3000 ppm or less, more preferably 15 ppm or more and 2500 ppm or less, further preferably 20 ppm or more and 2000 ppm or less, based on the total amount of the spiroglycol and the (meth)acrylic acid ester, from the viewpoint of suppressing the polymerization of the (meth)acrylic acid ester.

The usage proportions of the N-oxyl compound and the polymerization inhibitor having a boiling point of 246°C or lower described above can be appropriately set depending on the desired performance and are not particularly limited. The usage proportions of the N-oxyl compound and the polymerization inhibitor having a boiling point of 246°C or lower are preferably 2:1 to 1:50, more preferably 1:1 to 1:40, further preferably 1:1.2 to 1:30, in terms of ppm from the viewpoint of suppressing polymerization in performing the transesterification reaction under a reflux condition.

In the production method of the present embodiment, an additional polymerization inhibitor other than the N-oxyl compound and the polymerization inhibitor having a boiling point of 246°C or lower described above may be further used in combination therewith. Examples of the additional polymerization inhibitor include, but are not particularly limited to: organic polymerization inhibitors such as hydroquinone (boiling point: 282°C), t-butylhydroquinone (boiling point: 288°C), 2-t-butyl-4,6-dimethylphenol (boiling point: 249°C), dibutylhydroxytoluene (boiling point: 265°C), 2,6-di-t-butyl-4-methylphenol (boiling point: 265°C), 2,4,6-tri-t-butylphenol (boiling point: 277°C), 4-t-butylcatechol (boiling point: 285°C), and phenothiazine (boiling point: 371°C); inorganic polymerization inhibitors such as copper chloride, copper sulfate, and iron sulfate; and organic salt-based polymerization inhibitors such as butyl dithiocarbamates and N-nitroso-N-phenylhydroxylamine aluminum salt. One of these additional polymerization inhibitors may be used alone, or two or more thereof may be used in combination. In the transesterification reaction, the polymerization inhibitors may be added in one portion of the desired usage amounts or may be added in divided portions. Also, the polymerization inhibitors may be continuously added via rectification columns.

In the transesterification reaction, it is desirable to remove or inactivate the catalyst, if necessary. Examples of the method for removing the catalyst include operations such as solid-liquid separation, filtration, centrifugation, hydrolysis treatment by the addition of water, extraction with an aqueous solution containing an acid or an alkali, crystal precipitation by the addition of a poor solvent, decrease in temperature, or concentration under reduced pressure, and adsorption. These operations can be appropriately selected depending on the types of the starting materials used, the type of the catalyst, the type of the resulting (meth)acrylate, reaction conditions, and the like. These operations may be used singly or in combination of two or more thereof. The method for inactivating the catalyst can involve adding water if the catalyst is, for example, metal alkoxide.

After the transesterification reaction described above, di(meth)acrylic acid ester of spiroglycol (spiroglycol di(meth)acrylate) is obtained as the (meth)acrylate compound of interest. In this respect, mono(meth)acrylic acid ester of spiroglycol (spiroglycol mono(meth)acrylate) may be secondarily produced.

The production method of the present embodiment, as mentioned above, produces highly pure spiroglycol di(meth)acrylate with good reproducibility at an industrial level, as compared with conventional techniques.

The rate of conversion can be appropriately set depending on the desired performance and is not particularly limited. The rate of conversion is preferably 98.5% by mass or more, more preferably 99.0% by mass or more, further preferably 99.5% by mass or more, based on the mass of the charged spiroglycol from the viewpoint of industrial productivity or the like.

The rate of selection can be appropriately set depending on the desired performance and is not particularly limited. The rate of selection is preferably 97.0% by mass or more, more preferably 97.5% by mass or more, further preferably 98.0% by mass or more, based on the mass of the charged spiroglycol from the viewpoint of industrial productivity or the like.

The yield can be appropriately set depending on the desired performance and is not particularly limited. The yield is preferably 95.5% by mass or more, more preferably 96.5% by mass or more, further preferably 97.5% by mass or more, from the viewpoint of industrial productivity or the like.

The production method of the present embodiment may comprise the step of purifying the obtained product after performing the transesterification reaction as described above. The purification step can be the step of removing an unreacted reaction product and/or a by-product. The purification step may appropriately employ a method that is generally used as a resin purification method. Specific examples thereof include a reprecipitation method of dissolving the (meth)acrylate compound in a solvent, followed by dropwise addition to a poor solvent or water, a liquid-liquid extraction method, and distillation and purification. The purification step may employ liquid-liquid extraction using acetone, toluene, or an aqueous sodium carbonate solution.

### [Application]

The (meth)acrylate compound obtained by the production method of the present embodiment is useful as an intermediate or a monomer of polymer materials such as polycarbonate, polyester, polyester carbonate, polyacrylate, polyurethane, polyether polyol, and epoxy resin, and further as a starting material for coating materials, ink, adhesives, plasticizers, lubricating oils, films, sheets, and the like.

A (meth)acrylate-containing composition obtained by the production method of the present embodiment may be used alone or may be used in combination with an additional resin starting material or resin, if necessary. Examples of the additional resin starting material include compounds such as other (meth)acrylates, urethane (meth)acrylate, alcohols, thiol, and polyamic acid. Examples of the additional resin include polycarbonate, polyester, polyester carbonate, polyacrylate, polyurethane, polyether polyol, epoxy resin, polyimide, silicone resin, alicyclic hydrocarbon resin, and hydrocarbon resin and further include these resins terminally modified with a (meth)acrylic group, a vinyl group, an acid, ester, halogen, or the like, though the resin is not limited thereto.

The usage method thereof is not limited, and the (meth)acrylate-containing composition may be added before or after synthesis of the resin starting material or the resin used in combination therewith. Specifically, the (meth)acrylate-containing composition can be mixed with the resin starting material or the resin used in combination therewith to prepare a resin composition. In this respect, the resin composition may be formed by melting by heating, or curing may be allowed to progress at this point in time. Glass or film surface is coated with the obtained resin composition, which is then photocured, for example, to obtain a cured product. The obtained resin composition may be dissolved in any of various solvents to prepare a solution, with which glass or film surface can then be coated, followed by drying and photocuring. Alternatively, another resin cured product may be dipped in the (meth)acrylate-containing composition of the present embodiment for impregnation.

The (meth)acrylate-containing composition may be used in combination with any of various photoreaction initiators, may be used in combination with inorganic matter such as oxide particles, may be used in combination with semiconductor particles, may be used in combination with a pigment, may be used in combination with a viscosity adjuster such as rosin, or may be used in combination with a surfactant. Alternatively, the (meth)acrylate-containing composition may be added as a crosslinking agent or a compatibilizing agent into other resins.

The (meth)acrylate compound obtained by the production method of the present embodiment is useful as a polymerizable compound for thermosetting resin compositions, photocurable resin compositions, ionizing radiation-curable ink compositions, hologram compositions, and the like, or as an additive for compositions for lithium ion-conducting electrolytes in a wide range of fields, for example, optimal members such as optical lenses, illumination members, electronic materials, and automobile members.

The resin composition comprising the (meth)acrylate-containing composition obtained by the production method of the present embodiment is useful as a starting material for coating materials, adhesives, films, sheets, and the like and can be used, in such application, as a component for an automobile, a component for construction, a vehicle window, various components (including optical members) for televisions/video cameras/audio players/smart phones/displays/computers/copiers, etc., a component for furniture such as illumination covers/window shades/interior equipment, optical elements such as light diffuser plates, light guide plates (light guides), optical lenses, optical molded products (including substrates for optical discs), and light-diffusing films, optical films, optical filters, protective films, transparent pressure-sensitive adhesive sheets, coating agents, LED/organic EL encapsulants, interlayer insulating materials for electronic components, resist ink such as solder resists for printing substrates, photocurable resin compositions for three-dimensional modeling, and the like.

### Examples

Hereinafter, the present invention will be described in more detail with reference to Examples and Comparative Examples. However, the technical scope of the present invention is not limited by these examples. In the description below, the term "%" denotes "% by mass", and the term "ppm" denotes "ppm by mass", unless otherwise specified.

### [Example 1]

### First step

A 300 ml flaks equipped with a stirrer, a thermometer, a gas inlet tube, and a rectification column (connected with a timer-controlled reflux head with a condenser) was charged with 36.49 g (0.12 mol) of SPG (spiroglycol), 93.73 g (1.09 mol) of MA (methyl acrylate), 0.76 g (2.67 mmol) of a catalyst TTIP (tetraisopropyl orthotitanate), and as polymerization inhibitors, 0.004 g (30.5 ppm based on the total amount of the charged SPG and MA) of MEHQ (4-methoxyphenol) and 0.0024 g (18.3 ppm based on the total amount of the charged SPG and MA) of 4H-TEMPO (4-hydroxy-2,2,6,6-tetramethylpiperidine-1-oxyl), and the liquid was bubbled with an oxygen-containing gas (3 vol% of oxygen and 97 vol% of nitrogen).

Transesterification reaction was carried out with stirring at a reaction liquid temperature of 80°C, and a mixed liquid of secondarily produced MeOH and the starting material MA was distilled off from the reaction system via the rectification column. MA having the same weight as that of the distillate was added to the reaction liquid as needed.

As a result of quantifying the amount of MeOH contained in the distillate from the reaction system, the reaction was completed after 4 hours from the start of stirring. Therefore, the heating of the reaction liquid was terminated.

The reaction liquid temperature was lower to 60°C or lower by cooling. Then, 4.64 g (0.26 mol) of H₂O was added thereto to inactivate the TTIP catalyst.

### Second step

The liquid temperature of the reaction product obtained in the first step was kept at 60°C by the application of heat, and H₂O and MA were then distilled off under reduced pressure. 130 g of acetone was added to the remaining reaction product, which was then dissolved by heating to 55°C, followed by filtration using a filtration aid (Radiolite #800).

After dissolution by reheating (55°C), the liquid temperature was lowered to room temperature by cooling to precipitate SPGDA (spiroglycol diacrylate). Crystals were collected.

The crystals were rinsed with an appropriate amount of acetone and then dried at 75°C.

### [Example 2]

### First step

A 300 ml flaks equipped with a stirrer, a thermometer, a gas inlet tube, and a rectification column (connected with a timer-controlled reflux head with a condenser) was charged with 36.08 g (0.12 mol) of SPG (spiroglycol), 89.53 g (1.04 mol) of MA (methyl acrylate), 0.56 g (1.97 mmol) of a catalyst TTIP (tetraisopropyl orthotitanate), and as polymerization inhibitors, 0.249 g (1,969 ppm based on the total amount of the charged SPG and MA) of MEHQ (4-methoxyphenol) and 0.0263 g (208.0 ppm based on the total amount of the charged SPG and MA) of 4H-TEMPO (4-hydroxy-2,2,6,6-tetramethylpiperidine-1-oxyl), and the liquid was bubbled with an oxygen-containing gas (3 vol% of oxygen and 97 vol% of nitrogen).

Transesterification reaction was carried out with stirring at a reaction liquid temperature of 80°C, and a mixed liquid of secondarily produced MeOH and the starting material MA was distilled off from the reaction system via the rectification column. MA having the same weight as that of the distillate was added to the reaction liquid as needed.

As a result of quantifying the amount of MeOH contained in the distillate from the reaction system, the reaction was completed after 4.8 hours from the start of stirring. Therefore, the heating of the reaction liquid was terminated.

The reaction liquid temperature was lower to 60°C or lower by cooling. Then, 4.63 g (0.26 mol) of H₂O was added thereto to inactivate the TTIP catalyst.

### Second step

The liquid temperature of the reaction product obtained in the first step was kept at 60°C by the application of heat, and H₂O and MA were then distilled off under reduced pressure. 130 g of acetone was added to the remaining reaction product, which was then dissolved by heating to 55°C, followed by filtration using a filtration aid (Radiolite #800).

After dissolution by reheating (55°C), the liquid temperature was lowered to room temperature by cooling to precipitate SPGDA (spiroglycol diacrylate). Crystals were collected.

The crystals were rinsed with an appropriate amount of acetone and then dried at 75°C.

### [Comparative Example 1]

A 300 ml flaks equipped with a stirrer, a thermometer, a gas inlet tube, and a rectification column (connected with a timer-controlled reflux head with a condenser) was charged with 36.35 g (0.12 mol) of SPG (spiroglycol), 89.36 g (1.04 mol) of MA (methyl acrylate), 0.69 g (2.43 mmol) of a catalyst TTIP (tetraisopropyl orthotitanate), and 0.20 g (1,580 ppm based on the total amount of the charged SPG and MA) of a polymerization inhibitor HQ (hydroquinone), and the liquid was bubbled with an oxygen-containing gas (3 vol% of oxygen and 97 vol% of nitrogen).

Transesterification reaction was carried out with stirring at a reaction liquid temperature of 80°C, and a mixed liquid of secondarily produced MeOH and the starting material MA was distilled off from the reaction system via the rectification column. Precipitates were confirmed in the reaction liquid before the completion of reaction. Therefore, the reaction was discontinued, and the precipitates were compositionally analyzed by SEM-EDS and were consequently confirmed to be a MA polymer having an element ratio of C:O = 6:4.

### [Comparative Example 2]

A 300 ml flaks equipped with a stirrer, a thermometer, a gas inlet tube, and a rectification column (connected with a timer-controlled reflux head with a condenser) was charged with 36.63 g (0.12 mol) of SPG (spiroglycol), 89.36 g (1.04 mol) of MA (methyl acrylate), 0.80 g (2.81 mmol) of a catalyst TTIP (tetraisopropyl orthotitanate), and 0.0028 g (22.1 ppm based on the total amount of the charged SPG and MA) of a polymerization inhibitor 4H-TEMPO (4-hydroxy-2,2,6,6-tetramethylpiperidine-1-oxyl), and the liquid was bubbled with an oxygen-containing gas (3 vol% of oxygen and 97 vol% of nitrogen).

Transesterification reaction was carried out with stirring at a reaction liquid temperature of 80°C, and a mixed liquid of secondarily produced MeOH and the starting material MA was distilled off from the reaction system via the rectification column. Precipitates were confirmed in the reaction liquid before the completion of reaction. Therefore, the reaction was discontinued, and the precipitates were compositionally analyzed by SEM-EDS and were consequently confirmed to be a MA polymer with C:O = 6:4.

### [Comparative Example 3]

A 300 ml flaks equipped with a stirrer, a thermometer, a gas inlet tube, and a rectification column (connected with a timer-controlled reflux head with a condenser) was charged with 36.01 g (0.12 mol) of SPG (spiroglycol), 92.10 g (1.07 mol) of MA (methyl acrylate), 0.80 g (2.81 mmol) of a catalyst TTIP (tetraisopropyl orthotitanate), and 0.171 g (1,325 ppm based on the total amount of the charged SPG and MA) of a polymerization inhibitor MEHQ (4-methoxyphenol), and the liquid was bubbled with an oxygen-containing gas (3 vol% of oxygen and 97 vol% of nitrogen).

Transesterification reaction was carried out with stirring at a reaction liquid temperature of 80°C, and a mixed liquid of secondarily produced MeOH and the starting material MA was distilled off from the reaction system via the rectification column. Precipitates were confirmed in the reaction liquid before the completion of reaction. Therefore, the reaction was discontinued, and the precipitates were compositionally analyzed by SEM-EDS and were consequently confirmed to be a MA polymer having an element ratio of C:O = 6:4.

### [Table 1]

**Table 1**

| | | | Example 1 | Example 2 | Comparative Example 1 | Comparative Example 2 | Comparative Example 3 |
|---|---|---|---|---|---|---|---|
| Polymerization inhibitor | MQ | ppm | 30.5 | 1,969 | | | 1,325 |
| | HQ | | | | 1,580 | | |
| | 4H-TEMPO | | 18.3 | 208.0 | | 22.1 | |
| Presence or absence of precipitate | | | Absent | Absent | Present | Present | Present |
| Production efficiency | Rate of conversion | % | 100.0 | 100.0 | 99.8 | 100.0 | 69.8 |
| | Rate of selection | % | 98.0 | 98.8 | 94.4 | 95.8 | 56.0 |
| | Yield | % | 98.0 | 98.8 | 94.2 | 95.8 | 39.1 |

| | | | | | | | |
|---|---|---|---|---|---|---|---|
| In each Comparative Example, the reaction was discontinued because polymer precipitates were confirmed during the reaction. The production efficiency was described as a value obtained when the polymer was precipitated. | | | | | | | |

## Claims

1. A method for producing a (meth)acrylate compound, comprising at least the step of
performing transesterification reaction of spiroglycol and a (meth)acrylic acid ester in the presence of a polymerization inhibitor, wherein
the polymerization inhibitor comprises an N-oxyl compound and a polymerization inhibitor having a boiling point of 246°C or lower.

2. The method for producing a (meth)acrylate compound according to claim 1, wherein
in the step of performing the transesterification reaction, the transesterification reaction is performed in the absence of a solvent other than the (meth)acrylic acid ester.

3. The method for producing a (meth)acrylate compound according to claim 1, wherein
in the step of performing the transesterification reaction, a content ratio of the N-oxyl compound is 5 ppm or more and 300 ppm or less based on a total amount of the spiroglycol and the (meth)acrylic acid ester.

4. The method for producing a (meth)acrylate compound according to claim 1, wherein
in the step of performing the transesterification reaction, a content ratio of the polymerization inhibitor having a boiling point of 246°C or lower is 10 ppm or more and 3000 ppm or less based on a total amount of the spiroglycol and the (meth)acrylic acid ester.

5. The method for producing a (meth)acrylate compound according to claim 1, wherein
the polymerization inhibitor having a boiling point of 246°C or lower comprises a phenolic compound and/or a quinone compound.

6. The method for producing a (meth)acrylate compound according to claim 1, wherein
the polymerization inhibitor having a boiling point of 246°C or lower comprises hydroquinone monomethyl ether.

7. The method for producing a (meth)acrylate compound according to claim 1, wherein
the N-oxyl compound is one or more selected from the group consisting of 4-hydroxy-2,2,6,6-tetramethylpiperidine-1-oxyl, 4-oxo-2,2,6,6-tetramethylpiperidine-1-oxyl, 4-methoxy-2,2,6,6-tetramethylpiperidine-1-oxyl, 4-cyano-2,2,6,6-tetramethylpiperidine-1-oxyl, 4-amino-2,2,6,6-tetramethylpiperidine-1-oxyl, 4-carboxy-2,2,6,6-tetramethylpiperidine-1-oxyl, 4-methacryloyloxy-2,2,6,6-tetramethylpiperidine-1-oxyl, 4-acetamido-2,2,6,6-tetramethylpiperidine-1-oxyl, 4-maleimido-2,2,6,6-tetramethylpiperidine-1-oxyl, 4-phosphonooxy-2,2,6,6-tetramethylpiperidine-1-oxyl, 4-maleimido-2,2,6,6-tetramethylpiperidine-1-oxyl, pyrrolidine-1-oxyl free radical compounds, 3-carboxy-2,2,5,5-tetramethylpyrrolidine-1-oxyl, and 2,2,6,6-tetramethylpiperidine-1-oxyl.

8. The method for producing a (meth)acrylate compound according to claim 1, wherein
the (meth)acrylic acid ester is a (meth)acrylic acid alkyl ester.
